(19) Europäisches Patentamt
European Patent Office
Office européen des brevets

(11) Publication number: 0 124 380 B1

(12) EUROPEAN PATENT SPECIFICATION

(45) Date of publication of patent specification: 10.12.86

(51) Int. Cl.[4]: C 07 C 57/04, C 07 C 51/353, C 07 C 67/343, C 07 C 69/54

(21) Application number: 84302924.0

(22) Date of filing: 01.05.84

# (54) Preparation of unsaturated acids and esters.

(30) Priority: 02.05.83 US 490908

(43) Date of publication of application: 07.11.84 Bulletin 84/45

(45) Publication of the grant of the patent: 10.12.86 Bulletin 86/50

(84) Designated Contracting States: DE FR GB SE

(56) References cited:
EP-A-0 055 534
DE-A-1 294 956
GB-A-1 207 415
GB-A-1 207 416
GB-A-1 573 272

(73) Proprietor: THE STANDARD OIL COMPANY
Midland Building
Cleveland, Ohio 44115 (US)

(72) Inventor: Guttmann, Andrew Tytus
5241 Philip Street
Maple Heights, OH 44137 (US)
Inventor: Grasselli, Robert Karl
150 Greentree Road
Chagrin Falls, OH 44022 (US)

(74) Representative: Smith, Sydney et al
Elkington and Fife High Holborn House 52/54
High Holborn
London WC1V 6SH (GB)

EP 0 124 380 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to an improved process for the production of unsaturated carboxylic acids or esters.

The condensation reaction of saturated carboxylic acids and esters with carbonyl compounds to produce unsaturated acids and esters is known and various catalysts for this reaction are known. Such condensations are usually carried out by passing the vaporized reactants alone or with an inert carrier gas over a catalyst and are not usually oxygen-promoted. However, DE—A—1 294 956 discloses a process for the production of methyl acrylate by oxidation of methyl acetate, or of a mixture of methanol and methyl acetate, followed by condensation of formaldehyde with methyl acetate, in the gaseous phase, in the presence of oxygen, using a catalyst consisting of 15—40% $TiO_2$, 20—40% $V_2O_5$ and 20—65% (ortho)phosphoric acid by weight. U.S. Patent No. 4,165,438 discloses the production of acrylic acids and esters by reacting a lower alkanoic acid or a lower alkyl ester thereof with formaldehyde in the presence of a vanadium orthophosphate catalyst.

U.S. Patent No. 4,339,598 and 4,324,908 disclose vanadium-antimony catalysts and promoted phosphate catalysts, respectively, as being effective for the vapour phase condensation of saturated monocarboxylic acids or esters with formaldehyde, to produce unsaturated carboxylic acids or esters.

It has now been discovered that over certain condensation catalysts, in the condensation of formaldehyde or formaldehyde derivatives with saturated acids or esters, the product yields of corresponding unsaturated acids or esters are greatly improved when the reaction is conducted in the presence of oxygen. This advance over the art significantly increases the economic incentive for the production of important monomers, such as methyl acrylate, from starting materials easily available from synthesis gas, such as methanol, formaldehyde, acetic acid and methyl acetate.

### Summary of the invention

Greatly improved yields of unsaturated acids and esters are obtained when the condensation of formaldehyde, or its derivatives, with saturated monocarboxylic acids or esters over certain condensation catalysts is conducted in the presence of oxygen.

### Detailed description of invention

The present invention is an improved process for the catalytic vapor-phase condensation of formaldehyde, or formaldehyde derivatives, with saturated monocarboxylic acids or esters, by conducting the reaction in the presence of oxygen. This oxygen-promoted condensation reaction is conducted similarly to a condensation reaction which is not oxygen-promoted. The reactant mixture is vaporized and the vapors passed along with oxygen or an oxygen-containing gas over a particular catalyst in a reaction zone.

The reactant mixture consists of formaldehyde, or its derivatives, and carboxylic acids or their derivatives. Formaldehyde in an aqueous solution may be used. Any formaldehyde derivative capable of undergoing a condensation reaction with a carboxylic acid or its derivative may be used. The preferred formaldehyde derivatives are methylal and trioxane.

Any saturated monocarboxylic acid or its ester capable of undergoing a condensation reaction with formaldehyde or formaldehyde derivatives can be used. The preferred acids and esters are acetic acid and acetates and propionic acid and propionates. A preferred aspect of the invention is to prepare acrylic acid and methacrylic acid derivatives from formaldehyde and acetic or propionic acid and their esters.

After the reactants are mixed in the desired ratio, the mixture is vaporized and the vapors passed along with oxygen or an oxygen containing gas over the chosen catalyst. Air is a convenient source of oxygen. Additional diluents such as nitrogen or steam may be used.

The molar ratio of the acid or acid derivative to methylal or formaldehyde may range from 1:10 to 10:1. The preferred molar ratio is from 2:1 to 10:1. The molar ratio of oxygen to methylal or formaldehyde can be from 0.2:1 to 10:1 with the preferred ratio being from 3:1 to 6:1.

The reaction temperature may range from 200°C to about 500°C, preferably from 300°C to 450°C. The average residence time may be from 2 to about 60 seconds, but a residence time of from 3 to 15 seconds is preferred.

The improved results are obtained with catalysts selected from among those described in US Patents No. 4,324,908 and 4,339,598, herein incorporated by reference. Thus, mixed iron phosphates promoted with molybdenum or compositions containing vanadium and antimony give excellent results.

Catalysts for use in the process according to the invention are those having the epirical formulas:

a) $VSb_mO_x$

wherein

m=0.5—40; and

x is determined by the nature and oxidation state of the other elements;

b) $VSb_mA_aB_bC_cO_x$

wherein

m=0.5—40; and

A=alkali metal, alkaline earth metal, Tl, La, rare earth metal, Th, or mixtures thereof,

B=Cu, Ag, Fe, Co, Ni, Mn, Cr, Nb, Ta, Ti, P, As, Sn, B, U or mixtures thereof;
C=Mo, W, Te, Bi, or mixtures thereof;
a=0—1;
b=0—1;
c=0—1; and
x is determined by the nature and oxidation state of the other elements; and
c) $A_aB_bC_cPO_x$

wherein
A is Fe, Ni, Co, Mn, Cu, Ag or mixtures thereof;
B is alkali metal, alkaline earth metal, Tl or mixtures thereof;
C is Se, Y, La, rare earth metal, Th, Nb, Mo, Te, Cr, Ta, As, Sb, U or mixtures thereof; and
a=0.2—3.0;
b=0—1.5;
c=0—2.0; and
x is determined by the nature and oxidation state of the other elements.

Other suitable catalysts include the following: molybdates, tungstates, vanadates and chromates of various transition elements, optionally promoted with alkali- and alkaline earth metals; heteropoly acids of molybdenum and tungsten, and their salts; crystalline alumino-silicates including zeolites X and Y, mordenites, the ZSM-series, and the like, suitably exchanged and/or impregnated with alkali metals and other elements such as Fe, V, Sb, Pt, Ag, Mo, or W.

The catalysts may be used unsupported, but the use of a suitable carrier, such as silica, alumina, amorphous silica-alumina, mixtures of silica and alumina, titania, zirconia, natural clays, and the like is preferred. The concentration of the active catalyst on the support may range from about 10% to about 80% by weight.

## Specific embodiments

### Example 1

A catalyst having the composition 50% $K_{0.4}$ $Cr_{0.1}$ $Fe_{0.47}$ $Mo_{0.1}$ $PO_x$+50% $Al_2O_3$ was prepared according to the procedure given in U.S. Patent No. 4,324,908 at column 3, incorporated by reference. Five cc of the catalyst was charged to a fixed-bed microreactor equipped with a preheat leg serving as a vaporizer and immersed in a temperature-controlled salt bath at 350°C. In *Experiment A*, liquid feed consisting of a mixture of methyl acetate and an aqueous formaldehyde solution in a molar ratio of methyl acetate/formaldehyde/water=10/1/3 was injected by a syringe pump into the reactor, through the preheat leg, over a period of 70 minutes, at such a rate that the feed vapors passed over the catalyst at an average contact time of 5 seconds. The reactor effluent was condensed, weighed and analyzed by gas chromatography. The condensation products were methyl acrylate and acrylic acid. The combined per pass conversion (ppc) to these condensation products, based on formaldehyde charged, was 33%.

In *Experiment B*, using a fresh batch of catalyst, the same liquid feed was injected into the reactor, but with air added as a carrier gas. The molar ratios were methyl acetate/formaldehyde/water/air=10/1/3/23.8; the molar ratio of oxygen to formaldehyde was 5:1; the temperature was 350°C and the average residence time of the total feed was 5 seconds. The combined per pass conversion to methyl acrylate and acrylic acid, based on formaldehyde charged, was 75%.

As can be seen from the summary of results in Table I, the results of experiments A and B show the improvement of the condensation yield when air is added to the feed.

### Example 2—4

In Examples 2—4, methylal, $CH_2(OCH_3)_2$, served as a formaldehyde source. The condensations were carried out with methyl acetate and with methyl propionate. In each example, one condensation reaction was carried out with nitrogen as an inert carrier gas and another reaction with air. The molar ratios were: ester/methylal/$N_2$ (or air)=10/1/22. In the case of air as a carrier gas, the $O_2$/methylal ratio was 4.6:1. The temperature was 350°C; the average residence time of the total feed was 4.3 seconds. The catalyst of Example 2 was prepared according to the procedure disclosed in U.S. Patent No. 4,339,598 at column 3, previously incorporated by reference. The catalysts of Examples 3 and 4 were prepared as indicated in Example 1. The results are summarized in Table II wherein each Example shows the percentage (%) per pass conversion (ppc) to condensation products obtained, based on methylal charged, utilizing the inert nitrogen carrier gas as compared to reaction results obtained utilizing oxygen. The results clearly show that in the presence of oxygen, the yield of the condensation products is greatly increased.

TABLE I

Example 1—Condensation of methyl acetate with formaldehyde effect of air vs. nitrogen

| Experiment | Reactants (molar ratio) | Carrier gas | % ppc of total condensation products |
|---|---|---|---|
| A | methyl acetate/formaldehyde/water 10/1/3 | — | 33 |
| B | methyl acetate/formaldehyde/water/air 10/1/3/23.8 | Air | 75 |

TABLE II

Condensation of methylal with methyl acetate and methyl propionate effect of air vs. nitrogen

| Example | Catalyst | Ester | Carrier gas | % ppc total condensation products |
|---|---|---|---|---|
| 2 | 42% $VSb_5O_x+42\%Al_2O_3+16\%SiO_2$ | Methyl Acetate | $N_2$<br>AIR | 43.6[(1)]<br>82.9 |
| 3 | 50% $K_{0.2}Mn_{0.25}Fe_{0.49}Mo_{0.1}PO_x+50\%Al_2O_3$ | Methyl Acetate | $N_2$<br>AIR | 41.1[(1)]<br>100.1 |
| 4 | 50% $K_{0.2}Cr_{0.1}Fe_{0.47}Mo_{0.1}PO_x+50\%Al_2O_3$ | Methyl Propionate | $N_2$<br>AIR | 49.7[(2)]<br>67.4 |

(1) Methyl Acrylate+Acrylic Acid
(2) Methyl Methacrylate+Methacrylic Acid

**Claims**

1. A process for producing unsaturated carboxylic acids and esters comprising passing into a reaction zone, a vaporous mixture of a saturated monocarboxylic acid or its ester with formaldehyde or a formaldehyde derivative in the presence of oxygen over a condensation catalyst, characterised in that said catalyst is one of, or a mixture of (a), (b) and (c) wherein

(a) is

$$VSb_mO_x$$

wherein

m=0.5—40; and

x is determined by the nature and oxidation state of the other elements.

(b) is

$$VSb_mA_aB_bC_cO_x$$

wherein

m=0.5—40; and

A=alkali metal, alkaline earth metal, Tl, La, rare earth metal, Th, or mixtures thereof,

B=Cu, Ag, Fe, Co, Ni, Mn, Cr, Nb, Ta, Ti, P, As, Sn, B, U or mixtures thereof,

C=Mo, W, Te, Bi, or mixtures thereof;

a=0—1;

b=0—1;

c=0—1; and

x is determined by the nature and oxidation state of the other elements; and

(c) is

$$A_aB_bC_cPO_x$$

wherein

A is Fe, Ni, Co, Mn, Cu, Ag or mixtures thereof;

B is alkali metal, alkaline earth metal, Tl or mixtures thereof;

C is Se, Y, La, rare earth metal, Th, Nb, Mo, Te, Cr, Ta, As, Sb, U or mixtures thereof; and
a=0.2—3.0;
b=0—1.5;
c=0—2.0; and
x is determined by the nature and oxidation state of the other elements.

2. A process as claimed in claim 1 characterized in that the ester is methyl acetate or methyl propionate.

3. A process as claimed in claim 1 characterized in that the formaldehyde derivative is methylal or trioxane.

4. A process as claimed in any of claims 1 to 3 characterized in that the saturated monocarboxylic acid is acetic acid or propionic acid.

5. A process as claimed in claim 1 characterized in that m in catalyst (b) is 3—15.

6. A process as claimed in claim 1 characterized in that the catalyst is on a support comprising silica and alumina.

7. A process as claimed in claim 2 characterized in that the ester is methyl acetate, the formaldehyde derivative is methylal.

8. A process for producing methyl acrylate and acrylic acid comprising passing into a reaction zone, a vaporous mixture of methyl acetate and formaldehyde, in the presence of oxygen at a temperature of from 300°C to 400°C in the presence of a catalyst comprising

$$A_aB_bC_cPO_x$$

wherein
A is Fe, Ni, Co, Mn, Cu, Ag or mixtures thereof;
B is alkali metal, alkaline earth metal, Tl or mixtures thereof;
C is Se, Y, La, rare earth metal, Th, Nb, Mo, Te, Cr, Ta, As, Sb, U or mixtures thereof; and
a=0.2—3.0;
b=0—1.5;
c=0—2.0; and
x is determined by the nature and oxidation state of the other elements.

9. A process for producing methyl acrylate and acrylic acid as in claim 8, but wherein the catalyst comprises

$$VSb_mA_aB_bC_cO_x$$

wherein
m=0.5—40; and
A=alkali metal, alkaline earth metal, Tl, La, rare earth metal, Th, or mixtures thereof,
B=Cu, Ag, Fe, Co, Ni, Mn, Cr, Nb, Ta, Ti, P, As, Sn, B, U or mixtures thereof,
C=Mo, W, Te, Bi, or mixtures thereof;
a=0—1;
b=0—1;
c=0—1; and
x is determined by the nature and oxidation state of the other elements.

10. A process for producing methyl methacrylate and methacrylate acid, in the conditions defined in claims 8 and 9, the vaporous reaction mixture consisting of methyl propionate and methylal.

**Patentansprüche**

1. Verfahren zur Herstellung von ungesättigten Carbonsäuren und Estern durch Einleiten eines dampfförmigen Gemisches, einer gesättigten Mono-Carbonsäure oder ihres Esters mit formaldehyd oder einem Formaldehydderivat in der Gegenwart von Sauerstoff über einen Kondensationskatalysator in eine Reaktionszone, dadurch gekennzeichnet, daß der Katalysator einer von oder ein Gemisch von (a), (b) und (c) ist, wobei

(a) $VSb_mO_x$ ist

wobei m=0,5 bis 40 ist; und
x bestimmt wird durch die Eigenart und den Oxidationszustand der anderen Elemente.

(b) $VSb_mA_aB_bC_cO_x$ ist

wobei m=0,5 bis 40 ist; und
A=Alkalimetall, Erdalkalimetall, Tl, La, Seltene Erdmetalle, Th oder Gemische davon,
B=Cu, Ag, Fe, Co, Ni, Mn, Cr, Nb, Ta, Ti, P, As, Sn, B, U oder Gemische davon,
C=Mo, W, Te, Bi oder Gemische davon;
a=0 bis 1;
b=0 bis 1;
c=0 bis 1; und

x bestimmt wird durch die Eigenwart und den Oxidationszustand der anderen Elemente; und

(c) $A_aB_bC_cPO_x$ ist

wobei
A: Fe, Ni, Co, Mn, Cu, Ag oder Gemische davon ist;
B: Alkalimetall, Erdalkalimetall, Tl oder Gemische davon ist;
C: Se, Y, La, Seltene Erdmetalle, Th, Nb, Mo, Te, Cr, Ta, As, Sb, U oder Gemische davon ist; und
a=0,2 bis 3,0;
b=0 bis 1,5;
c=0 bis 2,0; und
x bestimmt wird durch die Eigenart und den Oxidationszustand der anderen Elemente.

2. Verfahren nach Anspruch 1, dadurch gekennzeichnet, daß der Ester Methylacetat oder Methylpropionat ist.

3. Verfahren nach Anspruch 1, dadurch gekennzeichnet, daß das Formaldehydderivat Methylal oder Trioxan ist.

4. Verfahren nach einem der Ansprüche 1 bis 3, dadurch gekennzeichnet, daß die gesättigte Monocarbonsäure Essigsäure oder Propionsäure ist.

5. Verfahren nach Anspruch 1, dadurch gekennzeichnet, daß m im Katalysator (b) 3 bis 15 ist.

6. Verfahren nach Anspruch 1, dadurch gekennzeichnet, daß der Katalysator auf einem Träger ist der Siliziumdioxid und Aluminium umfaßt.

7. Verfahren nach Anspruch 2, dadurch gekennzeichnet, daß der Ester Methylacetat ist, das Formaldehydderivat Methylal ist.

8. Verfahren zur Herstellung von Methylacrylat und Acrylsäure durch Einleiten eines dampfförmigen Gemisches aus Methylacetat und Formaldehyd in der Anwesenheit von Sauerstoff bei einer Temperatur von 300°C bis 400°C in eine Reaktionszone in der Anwesenheit eines Katalysators, der umfaßt

$$A_aB_bC_cPO_x$$

wobei
A: Fe, Ni, Co, Mn, Cu, Ag oder Gemische davon ist;
B: Alkalimetall, Erdalkalimetall, Tl oder Gemische davon ist;
C: Se, Y, La, Seltene Erdmetalle, Th, Nb, Mo, Te, Cr, Ta, As, Sb, U oder Gemische davon ist; und
a=0,2 bis 3,0;
b=0 bis 1,5;
c=0 bis 2,0; und
x bestimmt wird durch die Eigenart und den Oxidationszustand der anderen Elemente.

9. Verfahren zur Herstellung von Methylacrylat und Acrylsäure nach Anspruch 8, wobei jedoch der Katalysator

$VSb_mA_aB_bC_cO_x$ umfaßt,

wobei
m=0,5 bis 40; und
A=Alkalimetall, Erdalkalimetall, Tl, La, Seltene Erdmetalle, Th oder Gemische davon ist,
B=Cu, Ag, Fe, Co, Ni, Mn, Cr, Nb, Ta, Ti, P, As, Sn, B, U oder Gemische davon,
C=Mo, W, Te, Bi oder Gemische davon;
a=0 bis 1;
b=0 bis 1;
c=0 bis 1; und
x bestimmt wird durch die Eigenart und den Oxidationszustand der anderen Elemente.

10. Verfahren zur Herstellung von Methylmethacrylat und Methacrylsäure unter den in Anspruch 8 und 9 definierten Bedingungen, wobei das dampfförmige Reaktionsgemisch aus Methylpropionat und Methylal besteht.

**Revendications**

1. Procédé de production d'acides et d'esters carboxyliques non saturés consistant à faire passer dans une zone de réaction un mélange à l'état de vapeur d'un acide monocarboxylique saturé ou de son ester avec du formaldéhyde ou un dérivé du formaldéhyde, en présence d'oxygène et sur un catalyseur de condensation, caractérisé en ce que ledit catalyseur est l'un des (a), (b) et (c), ou un mélange de ceux-ci,
(a) étant

$$VSb_mO_x$$

m=0,5—40; et

x est déterminé par la nature et l'état d'oxydation des autres éléments;
(b) étant

$$VSb_mA_aB_bC_cO_x$$

dans laquelle
m=0,5—40; et
A=métal alcalin, métal alcalino-terreux, Tl, La, métal des terres rares, Th ou des mélanges de ceux-ci,
B=Cu, Ag, Fe, Co, Ni, Mn, Cr, Nb, Ta, Ti, P, As, Sn, B, U ou des mélanges de ceux-ci,
C=Mo, W, Te, Bi, ou des mélanges de ceux-ci,
a=0—1,
b=0—1,
c=0—1, et
x est déterminé par la nature et l'état d'oxydation des autres éléments; et
(c) étant

$$A_aB_bC_cPO_x$$

dans laquelle
A est Fe, Ni, Co, Mn, Cu, Ag ou des mélanges de ceux-ci,
B est un métal alcalin, un métal alcalino-terreux, Tl ou des mélanges de ceux-ci,
C est Se, Y, La, un métal des terres rares, Th, Nb, Mo, Te, Cr, Ta, As, Sb, U ou des mélanges de ceux-ci,
a=0,2—3,0,
b=0—1,5,
c=0—2,0, et
x est déterminé par la nature et l'état d'oxydation des autres élémentes.

2. Procédé tel que revendiqué dans la revendication 1, caractérisé en ce que l'ester est de l'acétate de méthyle ou du propionate de méthyle.

3. Procédé tel que revendiqué dans la revendication 1, caractérisé en ce que le dérivé du formaldéhyde est du méthylal ou du troixanne.

4. Procédé tel que revendiqué dans l'une quelconque des revendications 1 à 3, caractérisé en ce que l'acide monocarboxylique saturé est l'acide acétique ou l'acide propionique.

5. Procédé tel que revendiqué dans la revendication 1, caractérisé en ce que m dans le catalyseur (b) vaut 3—15.

6. Procédé tel que revendiqué dans la revendication 1, caractérisé en ce que le catalyseur se trouve sur un support comprenant de la silice et de l'alumine.

7. Procédé tel que revendiqué dans la revendication 2, caractérisé en ce que l'ester est l'acétate de méthyle et le dérivé du formaldéhyde est le méthylal.

8. Procédé de production d'acrylate de méthyle et d'acide acrylique consistant à faire passer dans une zone de réaction un mélange à l'état de vapeur d'acétate de méthyle et de formaldéhyde, en présence d'oxygène à une température de 300°C à 400°C, en présence d'un catalylseur constitué par

$$A_aB_bC_cPO_x$$

dans laquelle
A est Fe, Ni, Co, Mn, Cu, Ag ou des mélanges de ceux-ci,
B est un métal alcalin, un métal alcalino-terreux, Tl ou des mélanges de ceux-ci,
C est Se, Y, La, un métal des terres rares, Th, Nb, Mo, Te, Cr, Ta, As, Sb, U ou des mélanges de ceux-ci,
a=0,2—3,0,
b=0—1,5,
c=0—2,0, et
x est déterminé par la nature et l'état d'oxydation des autres éléments.

9. Procédé de production d'acrylate de méthyle et d'acide acrylique de la manière indiquée dans la revendication 8, mais dans lequel le catalyseur est constitué par

$$VSb_mA_aB_bC_cO_x$$

dans laquelle
m=0,5—40; et
A=métal alcalin, métal alcalino-terreux, Tl, La, métal des torres rares, Th ou des mélanges de ceux-ci,
B=Cu, Ag, Fe, Co, Ni, Mn, Cr, Nb, Ta, Ti, P, As, Sn, B, U ou des mélanges de ceux-ci,
C=Mo, W, Te, Bi, ou des mélanges de ceux-ci,
a=0—1,
b=0—1,
c=0—1, et
x est déterminé par la nature et l'état d'oxydation des autres éléments.

10. Procédé de production de méthacrylate de méthyle et d'acide méthacrylique dans les conditions définies dans les revendications 8 et 9, le mélange de réaction à l'état de vapeur étant constitué de propionate de méthyle et de méthylal.